# EUROPEAN PATENT SPECIFICATION

(11) **EP 2 219 576 B1**
(45) Date of publication and mention of the grant of the patent: **02.01.2019**
(21) Application number: 08849984.3
(22) Date of filing: 13.11.2008
(51) Int. Cl.: A61F 13/15, A61F 13/00, A61F 13/08

(54) **ASSEMBLY FOR TREATING VENOUS ULCERS AND WOUNDS**
ANORDNUNG ZUR BEHANDLUNG VON VENENGESCHWÜREN UND WUNDEN
ENSEMBLE POUR LE TRAITEMENT D'ULCÈRES VEINEUX ET DE PLAIES

(30) Priority: 13.11.2007 US 987473 P; 21.03.2008 US 53117
(43) Date of publication of application: 25.08.2010
(73) Proprietor: Ravikumar, Sundaram, Briarcliff Manor, NY 10510 (US)
(72) Inventor: Ravikumar, Sundaram, Briarcliff Manor, NY 10510 (US)
(74) Representative: Davies, Gregory Mark
(86) International application number: PCT/US2008/083295
(87) International publication number: WO 2009/064822

(56) References cited:
- WO-A1-02/02181
- WO-A2-2006/114637
- US-A1- 2005 187 501
- US-A1- 2006 149 171
- US-A1- 2006 149 171
- US-A1- 2007 032 762

## Description

### BACKGROUND OF THE INVENTION

### 1. Field of the Invention

This invention relates to medical assemblies .. More particularly, this invention relates to mechanisms for applying pressure to a limb of the human body for healing purposes. While the invention has particular application in conjunction with the treatment of venous ulcers of the leg and possibly with other forms of medical treatment (e.g., sclerotherapy or vein stripping/removal for treatment for varicose veins) of the leg or other extremity, it is not limited thereto.

### 2. State of the Art

A venous ulcer is damage and loss of skin above the ankle that is the result of a problem with the veins in the leg. Venous ulcers typically develop on either side of the lower leg, above the ankle and below the calf. They are difficult to heal and often recur.

The veins of the leg are divided into the superficial and deep systems according to their position relative to the fascia.

The deep veins, which come together to form the popliteal and femoral veins lie within the fascia and are responsible for the venous return from the leg muscles. Dilated valveless sinusoids also lie within the fascia (more particularly in the soleus and gastrocnemius muscles). The sinusoids fill with blood when the leg is at rest.

The long saphenous vein which runs along the medial side of the leg from foot to groin and the short saphenous vein which runs at the back of the calf from foot to knee are the major vessels of the superficial venous system. These vessels lie outside the fascia and are responsible for the venous return from the skin and subcutaneous fat.

Communicating veins, sometimes called perforators because they perforate the deep fascia, join the two systems. The perforators, like the other veins in the leg, contain valves that permit the flow of blood in one direction only, from the outer or superficial system inwards to the deep veins.

The venous pressure at the ankle of a subject who is lying supine is around 10 mmHg, but on standing this will rise considerably due to an increase in hydrostatic pressure (equivalent to the weight of a vertical column of blood stretching from the point of measurement to the right auricle of the heart).

During walking, as the foot is dorsally flexed, the contraction of the calf muscle compresses the deep veins and soleal sinuses thereby emptying them of blood. As the foot is plantarly flexed, the pressure in the veins falls, the proximal valves close, and the veins are refilled by blood passing through the perforators from the superficial system. During this cycle, in a normal leg, the distal valves of the deep veins and the valves of the perforators will ensure that the expelled blood can go in only one direction - upwards, back to the heart.

Blockage or damage to the venous system will cause disruption to normal blood flow, which may manifest itself in a number of different ways according to the site and extent of the damage. If the valves in the superficial system are affected, venous return will be impaired and blood may accumulate in the veins causing them to become distended, leading to the formation of varicosities (varicose veins).

If the function of the perforator valves is impaired, the action of the calf muscle pump will tend to cause blood to flow in the reverse direction into the superficial system increasing the possibility of damage to the superficial vessels.

Following a deep vein thrombosis that results in complete or partial obstruction of a deep vein, the unrelieved pressure produced by the calf muscle pump on the perforator valves may cause these to become incompetent. If this occurs, there will be a large rise in the pressure in the superficial system, which may force proteins and red cells out of the capillaries and into the surrounding tissue. Here, the red cells break down releasing a red pigment that causes staining of the skin, an early indicator of possible ulcer formation.

Venous leg ulcers are generally shallow and red in color. The skin surrounding the ulcer is frequently discolored due to the staining described previously. Incompetent perforating vein valves can also cause malleolar venules to become dilated and appear as fine red threads around the ankle. This condition, called ankle flair, is also diagnostic of a venous ulcer.

US2006149171A1 relates to a method and apparatus for concurrent applications of intermittent pneumatic compression therapy and vacuum assisted closure therapy.

WO2006114637A2 relates to an apparatus for cleaning wounds.

### SUMMARY OF THE INVENTION

Viewed from a first aspect, there is provided an assembly comprising:
a) a pressure mechanism having a flexible member for attachment around a limb and an air chamber which assumes a first depressurized state and a second pressurized state, said air chamber having a length and a width, said width being less than half the width of said flexible member;
b) a pre-filled air bladder having a length and a width smaller than said width of said air chamber;
c) an absorbent foam, sponge or dressing coupled to the pre-filled air bladder; and
d) a suction conduit in fluid communication with the absorbent foam, sponge or dressing and adapted for coupling to a source of negative pressure;
wherein:
said pre-filled air bladder and said absorbent foam, sponge or dressing are adhered together as a unit, wherein said suction conduit is located between said absorbent foam, sponge or dressing and said pre-filled air bladder.

According to one aspect of the invention, the flexible member of the pressure mechanism is adapted to wrap around a leg or arm and over the pre-filled air bladder in order to secure the pre-filled air bladder and the foam, sponge or dressing to a wound or ulcer in the extremity. Thus, the flexible member is provided with some fixation structure such as a hook and loop closure mechanism. An air pumping mechanism is preferably coupled to the air chamber of the pressure mechanism in order to inflate the air chamber to a pressurized state. The air chamber of the pressure mechanism is preferably designed to apply pressure along a predefined area (e.g., the saphenous vein of a leg) as opposed to around an entire limb.

According to another aspect of the invention, the suction conduit is located either between the pre-filled air bladder and the absorbent foam, sponge or dressing which is adhered to the small air bladder, or the pre-filled air bladder is formed as a donut with a central opening and the suction conduit extends through the central opening. By coupling the suction conduit to a source of negative pressure, exudate from the wound or ulcer is sucked through the foam, sponge or dressing into the suction conduit.

Advantages of the invention will become apparent to those skilled in the art upon reference to the detailed description taken in conjunction with the provided figures.

### BRIEF DESCRIPTION OF THE DRAWINGS

**FIG. 1** is a broken schematic view of an assembly according to the invention.
**FIG. 2A** is an isometric view of a compression mechanism in accordance with the present invention, showing the mechanism in its unwrapped state.
**FIG. 2B** is an isometric view of an alternate compression mechanism in accordance with the present invention, showing the mechanism in its unwrapped state.
**FIG. 3**A is a view of an embodiment of a compression mechanism in accordance with the present invention which is intended to be wrapped around the lower leg for application of localized pressure to the lower leg in the vicinity of the short saphenous vein and which is shown in its unwrapped state with its body-contacting surface facing out of the page.
**FIG. 3B** is a view of an embodiment of a compression mechanism in accordance with the present invention which is intended to be wrapped around the upper leg (e.g., thigh) for application of localized pressure to the upper leg in the vicinity of the long saphenous vein and which is shown in its unwrapped state with its body-contacting surface facing into the page.
**FIGS. 4A-4C** are side, top cut-away, and cross-sectional views of an embodiment of a unit including a pre-filled air bladder, an absorbent foam, sponge or dressing, and a suction conduit where the suction conduit extends at least partially around the periphery of the unit.
**FIGS. 5A-5B** are broken exploded and side views of an embodiment of a unit including a pre-filled air bladder defining a central opening, an absorbent foam, sponge or dressing, and a suction conduit which extends into the central opening of the pre-filled air bladder.
**FIG. 6** is a broken exploded view of another embodiment of a unit including a pre-filled air bladder, an absorbent foam, sponge or dressing, and a suction conduit where the suction conduit extends into the center of the unit.
**FIGS. 7A-7B** are broken perspective views of a unit having straps for securing the unit in place.

### DETAILED DESCRIPTION OF THE PREFERRED EMBODIMENTS

For purposes herein, the terms "limb" and "extremity" are used interchangeably and are to be understood to include the arms, hands, legs, and feet.

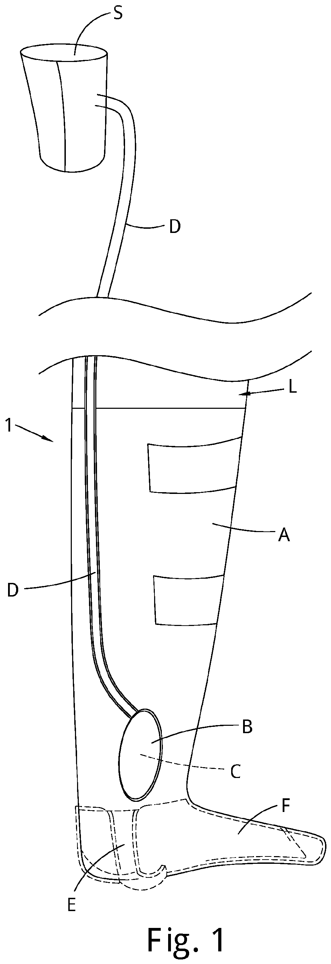

Turning to Fig. 1, a medical assembly 1 is provided. The medical assembly generally includes a compression mechanism A, a pre-filled air bladder B, an absorbent foam, sponge or dressing C, and a suction conduit D which is coupled to a suction apparatus S. The pre-filled air bladder B, absorbent foam, sponge or dressing C, and the suction conduit D may be provided as a unit. As shown, the compression mechanism A is wrapped around the leg L of a patient and includes an optional foot strap E which may be wrapped around the foot F of the patient. The suction conduit D is of desired length to couple the assembly 1 to the suction source S.

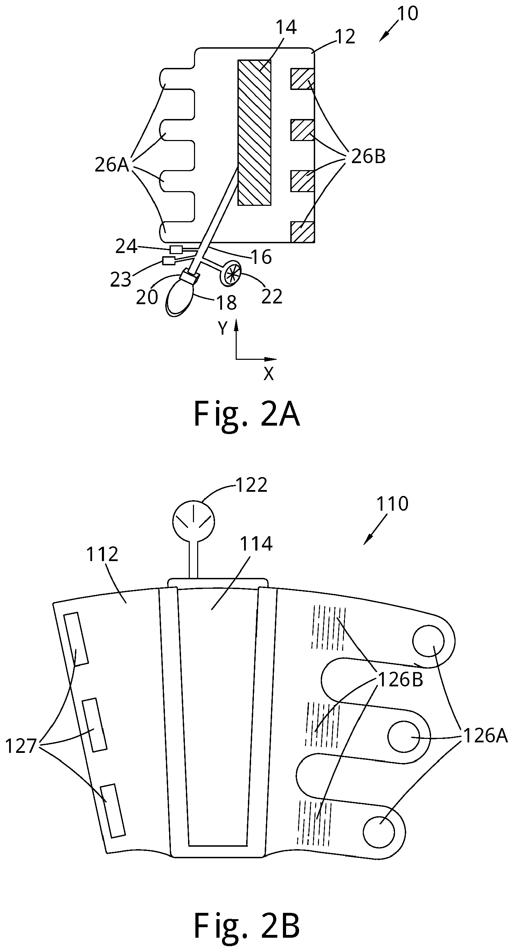

Turning to Fig. 2A, a first embodiment of compression mechanism (corresponding to compression mechanism A of Fig. 1) is provided for applying pressure to the lower leg of the human body. The compression mechanism 10 preferably includes a flexible wrap member 12 and one or more inflatable air bladder chambers 14 (preferably, a single air chamber as shown). The inflatable air bladder chamber 14 is preferably secured to the flexible wrap member 12 in its unwrapped state. For example, the flexible wrap member 12 may comprise two layers of elastomeric material with the air bladder chamber(s) 14 affixed between these two layers by nylon threads or other suitable fastening means. Alternatively, the flexible member 12 may include pockets into which the air bladder chamber(s) 14 are removably inserted and securely held therein. In yet another alternative embodiment, the air bladder may be glued or welded to the inside surface of the member 12. The elastomeric material of the member 12 may be realized from nylon, polyurethane, cotton, or other suitable material. A tube 16, which is in fluid communication with the air bladder chamber(s) 14, extends to a pumping bulb 18. The pumping bulb 18, which is preferably made of rubber, includes a valve 20 that regulates the pumping of air into the air bladder chamber(s) 14 via the tube 16. Air is pumped into the air bladder chamber(s) 14 by squeezing the pumping bulb 18. In this manner, the air bladder chamber(s) 14 are placed into a pressurized state. Preferably, a pressure gauge 22 is operably coupled to the air bladder chamber(s) 14 to provide a visual indication of the pressure level therein. An automatic pressure relief valve 23 and a manual pressure relief valve 24 may be operably coupled to the air bladder chamber(s) 14, for example via the tube 16. The automatic pressure relief valve 23 automatically vents the air in the chamber(s) 14 to the ambient environment when the internal pressure reaches a certain threshold maximum pressure. In the preferred embodiment, this threshold maximum pressure is between 30 to 40 mmHg, and most preferably around 40 mmHg; however it can be varied based upon the desired treatment. In this manner, the pressure inside the chamber(s) 14 cannot exceed the threshold maximum pressure, thereby reducing the danger of necrosis and other complications that arise from excessive pressure. The manual pressure relief valve 24 vents the air in the chamber(s) 14 to the ambient environment when manually actuated by the patient (or caregiver). In this manner, it facilitates quick and easy control over the internal pressure of the air chamber(s) 14. In alternative embodiments, the manual pressure relief valve 24 and possible the automatic relief valve 23 may be integrated into a common package.

The air chamber 14 is substantially longer in a first dimension (e.g., the Y dimension of FIG. 2A) than in a second dimension orthogonal thereto (e.g., the X dimension of FIG. 2A) such that the air chamber 14 can be positioned to extend substantially longitudinally along the lower leg to apply local pressure along its length (the Y dimension). Such local pressure is substantially constant along the length of the chamber 14. In the illustrative embodiment, the air chamber 14 is disposed such that it runs along the calf of the lower leg, which enables the air chamber 14 to apply local pressure to the short saphenous vein of the patient when securely wrapped around the patient's lower leg and inflated. However, the flexible member 12 and air chamber 14 may be adapted such that they are disposed along another portion of the lower leg (e.g., a portion of the leg below the knee), which enables the air chamber 14 to apply local pressure to such portion of the lower leg when inflated.

The flexible member 12 may include a strap (not shown) that extends around the heal (and/or other parts) of the foot when in use. This strap limits the upward travel of the flexible member 12 when in use. It may also have suspender hooks or slots (not shown) that allow for suspenders to be mated thereto which support the mechanism 10 by a band that wraps around the knee or thigh. The suspenders limit downward travel of the flexible member 12 when in use. These features reduce the travel of the flexible member 12 along the length of the leg such that its desired position is maintained during use.

In alternative embodiments, the flexible member 12 and air chamber 14 may be adapted such that they are disposed along a portion of the upper leg (e.g., a portion of the thigh), which enables the air chamber 14 to apply local pressure to such portion of the upper leg when inflated. For treatment of the upper leg, the flexible member 12 may define an opening (not shown) at the knee joint level to enable the patella (knee cap) to protrude therethrough. In this configuration, the flexible member 12 may extend below the knee joint level and securely wraps around portions of the lower leg to provide stability to the leg. It may also have suspender hooks or slots (not shown) that allow for suspenders to be mated thereto in order to support the mechanism 10 by a waist band when in use. The suspenders limit downward travel of the flexible member 12 when in use such that the flexible member 12 maintains its desired position.

Preferably, the flexible member 12 includes multiple hook and loop closure mechanisms 26A, 26B (e.g., VELCRO® members). In the preferred embodiment, the flexible member includes four hook and loop closure mechanisms as shown in FIG. 2A. These multiple closures enable the flexible member 12 and the air chamber(s) 14 to be securely wrapped around a portion of the human leg. If desired, other suitable fastening means may be used to secure the flexible member and the air chamber(s) to the human leg. For example, the flexible member may be adapted to form a sleeve-like shape with a zipper running along its length dimension. Alternatively, the zipper may be omitted such that sleeve-like flexible member is slid over the patient's leg until it is disposed in the desired position.

Preferably, pressure in the air chamber(s) is reduced/removed (e.g., the air chamber(s) are deflated) by user manipulation of the manual relief valve 24, and the pneumatic compression mechanism is removed from the leg by manually detaching the hook and loop closures and unwrapping the flexible member 12 from around the leg.

FIG. 2B illustrates an alternative compression mechanism 110 (corresponding to compression mechanism A of Fig. 1) which includes a flexible member 112 and one or more pre-inflated (pre-filled) and sealed air chambers 114 (preferably, a single air chamber as shown). The pre-inflated sealed air chamber 114 is preferably secured to the flexible member 112 in its unwrapped state. For example, the flexible member 112 may comprise two layers of elastomeric material with the air bladder chamber(s) 114 affixed between these two layers by nylon threads or other suitable fastening means. Alternatively, the flexible member 112 may include pockets into which the sealed air chamber(s) 114 are removably inserted and securely held therein. In yet another alternative embodiment, the air chamber may be glued or welded to the inside surface of the member 112. Regardless, a pressure gauge 122 is operably coupled to the air chamber(s) 114 to provide a visual indication of the pressure level therein. The flexible member 112 also includes multiple straps 125 having hook and loop closure mechanisms 126A, 126B (e.g., VELCRO® members), and multiple openings 127 for receiving the straps. In the preferred embodiment, the flexible member includes three straps 125 and three openings 127. In use, the straps are threaded through the openings and back on themselves such that the hook and loop closure mechanisms 126A and 126B engage each other. By pulling the straps tight and affixing them using the closure mechanisms, more or less pressure may be applied to the pre-filled chamber; thereby applying more or less pressure to the limb on which the compression mechanism is applied. The amount of pressure applied may be read by the pressure gauge 122 which may have numerical or other visible indications.

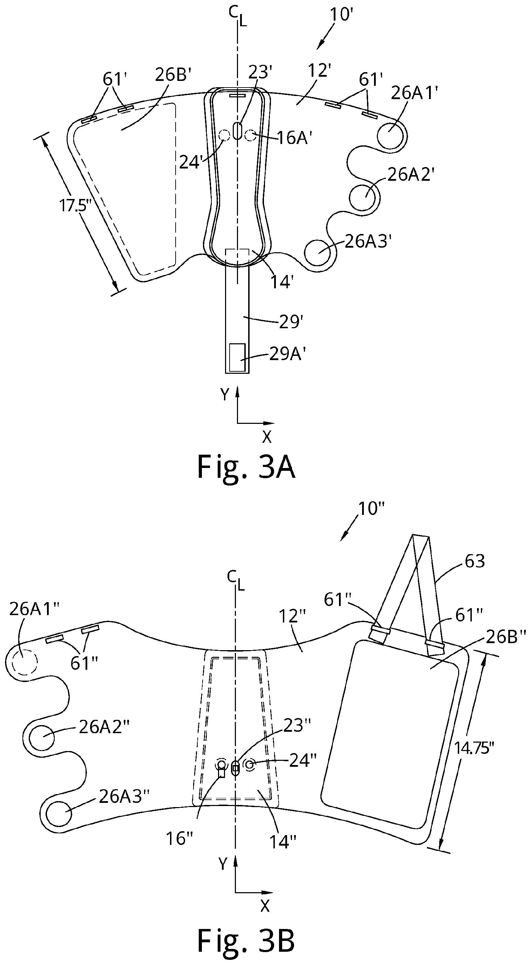

Turning now to FIG. 3A, an alternate embodiment of a pneumatic compression mechanism (corresponding to compression mechanism A of Fig. 1) is shown. The compression mechanism 10' includes a flexible member 12' and one or more inflatable air chambers 14' (preferably, a single air bladder as shown). The inflatable air chamber 14' may be formed from two walls that are bonded together, preferably by heat sealing, about a flange portion in a manner similar to the fluid-filled members 30 as described herein. The air chamber 14' is secured to the inside surface of the flexible member 12' preferably by gluing, welding or other suitable fastening means. Alternatively, the flexible member 12' may comprise two layers of elastomeric material with the air chamber(s) 14' affixed between these two layers by nylon threads or other suitable fastening means. The flexible member 12' is preferably realized from nylon, polyurethane, cotton, or other suitable material. A connector 16A' is in fluid communication with the air chamber(s) 14' via a port (not shown) which preferably extends through the bottom side of the air bladder chamber 14'. The connector 16A' mates to an inflation tube (not shown) for fluid connection to the pumping bulb as described above or other inflation mechanisms. An automatic pressure relief valve 23' and a manual pressure relief valve are in fluid communication with the air chamber(s) 14' via additional ports (not shown) which preferably extend through the bottom side of the air bladder chamber(s) 14'.

Preferably, the flexible member 12' includes multiple hook and loop closure mechanisms (e.g., VELCRO® members) which enable the flexible member 12' (and the air chamber(s) 14' secured thereto) to be securely wrapped around a portion of the human leg. In the exemplary embodiment of FIG. 2A, the flexible member 12' includes three hook buttons 26A1', 26A2', 26A3' disposed on the body-contacting side of member 12' that mate to a larger loop panel section 26B' disposed on the opposite side of member 12'. If desired, other suitable fastening means may be used to secure the flexible member 12' and the air bladder chamber(s) 14' to the human leg. For example, the flexible member 12' may be adapted to form a sleeve-like shape with a zipper running along its length dimension. Alternatively, the zipper may be omitted such that sleeve-like flexible member 12' is slid over the patient's leg until it is disposed in the desired position. The flexible member 12' may also include cut-outs (not shown) which provide enhanced flexibility of the member 12'.

During use, air is pumped into the air chamber(s) 14' by actuation of the pumping bulb (or other inflation mechanism). The air chamber 14' is substantially longer in a first dimension (e.g., the Y dimension of FIG. 3A) than in a second dimension orthogonal thereto (e.g., the X dimension of FIG. 3A) such that the air chamber 14' can be positioned to extend substantially longitudinally along the leg to apply local pressure along its length (the Y dimension). Such local pressure is substantially constant along the length of the chamber 14'.

In the exemplary embodiment shown, the air chamber 14' has a length of 13.00 inches (Y dimension) and width of 3.77 inches and 3.51 inches (X dimension) at its top and bottom ends, respectively, as shown. The width of the chamber 14' tapers as it extends away from the top and bottom ends to a minimal width, which is located relatively closer to the bottom end as shown. It will be appreciated that the air chamber 14' may take other shapes and sizes.

In another alternative embodiment as shown in FIG. 3B, a flexible member 12" and air chamber 14" of a compression mechanism 10" (corresponding to compression mechanism A of Fig. 1) are adapted such that they are disposed along a portion of the upper leg (e.g., a portion of the thigh), which enables the air chamber 14" to apply local pressure to such portion of the upper leg when inflated. The structure and operation of the elements of the mechanism 10" are analogous to the mechanism 10' as described above with respect to FIG. 3A and thus description of such elements (which are labeled with like numbers) are omitted for simplicity of description.

In the exemplary embodiment shown, the flexible member 12" is contoured to conform to the upper leg when wrapped around it. The air bladder chamber 14" has a length of 11.75 inches (Y dimension) and maximum width of 6.00 inches (X dimension) at its bottom end as shown. The width of the chamber 14" tapers as it extends away from the bottom end to the top end as shown. It will be appreciated that the air chamber 14" may take other shapes and sizes.

It will be appreciated that the chambers of either of the embodiments of FIGS. 3A and 3B may be pre-filled and sealed as described above with reference to FIG. 2B. It will also be appreciated that the air chambers which are arranged to receive air can be inflated using a pumping bulb (as shown in Fig. 2A), or can be inflated by an electric air pump (not shown) which can use batteries or AC wall current to pump air into the chamber(s).

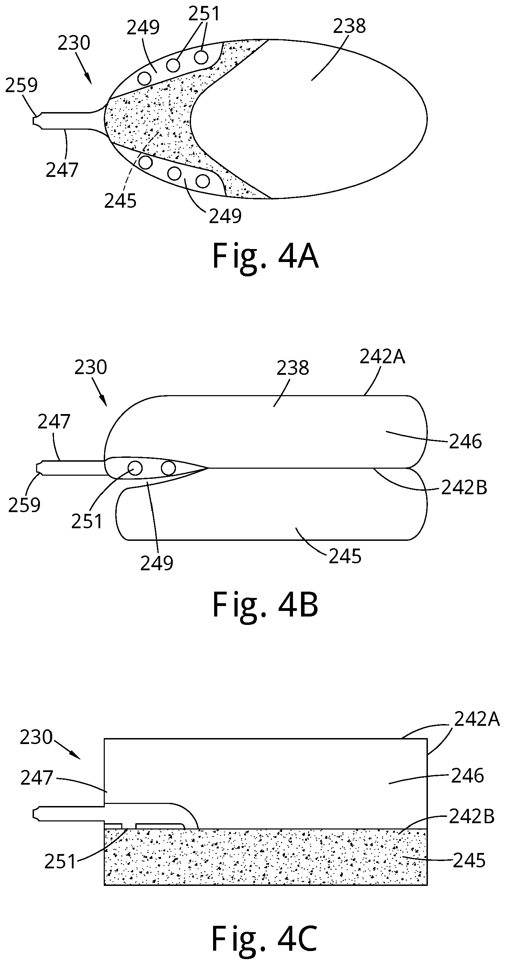

Turning now to FIGS. 4A-4C, a unit 230 for use in conjunction with the compression mechanisms 10, 10', 10", or 110 includes a sealed fluid-filled bladder 238, an absorbent foam, sponge or dressing 245, and a suction conduit 247 (corresponding to bladder B, foam, sponge or dressing C and at least a portion of suction conduit D of Fig. 1).

The fluid-filled member 238 may be formed in many of numerous ways. In one arrangement, the fluid-filled member includes two walls 242A, 242B that are bonded together, preferably by heat sealing, about a flange portion (not shown). The two walls define a chamber 246 therebetween that is filled with fluid. The fluid held in the chamber 246 can be a gas (such as air), a liquid (such as water), or a gel. The fluid inside the chamber 246 may be loaded with one or more therapeutic agents, such as antibiotics, growth factor, absorbents. In such configurations, the bottom wall 242B is realized from a semi-permeable material that allows the therapeutic agents retained in the chamber 246 to migrate through to the treatment site while maintaining the desired internal pressure in chamber 246. Such fluid might also be a gel compound that retains heat and/or cold such that is useful for hot and/or cold therapy of the treatment site

The fluid-filled member 238 preferably has an oval shape with a length on the order of four to six inches, a width on the order of two to four inches, and a height on the order of one-quarter to three-quarters of an inch. Enough fluid is preferably provided to prevent the walls of the member 238 from sagging and touching each other, although some sagging can be tolerated. The walls of fluid-filled member 238 may be formed from polyurethane, polyvinylchloride, nylon, or other plastic(s) known in the art and are of sufficient thickness (i.e., are sufficiently strong) such that the fluid-filled member 238 will not burst when 40mm Hg is applied to it by the compression mechanism. It will be appreciated that the fluid-filled member 238 may take other shapes and sizes, and may be formed from other materials.

Attached to the fluid-filled member 238 is the absorbent foam, sponge or dressing 245 (hereinafter referred to as "the sponge"). The sponge 245 has the ability to absorb exudate from the wound or ulcer. In addition, the sponge preferably has an open-cell structure which aids in wicking the exudate from the wound or ulcer. The sponge 245 is preferably of a similar size and shape as the fluid-filled member. The sponge 245 is preferably fixed by adhesive to the bottom wall 242B of the fluid-filled member such that that the sponge 245 and the fluid-filled member 238 cannot be easily separated from each other. Alternatively, the sponge may be lightly affixed to the fluid-filled member 242B by an adhesive film such that the sponge may be peeled off and replaced. As another alternative, the fluid-filled member 238 may be provided with a circumferential holding flap for the sponge 245 which may be inserted and into and removed from the flap as desired. If desired, the circumference of the sponge may be sealed either by injecting sealant into the sponge or by collapsing the open-cell structure of the sponge. Sealing the circumference of the sponge can help reduce air from entering the sponge when suction is applied to the unit as described below.

In the unit 230 of Figs. 4A-4C, the suction conduit 247 is provided in between the fluid-filled member 238 and the sponge 245. More particularly, suction conduit 247 is a tube having a bifurcated distal end 249 which defines a series of holes 251. The holes 251 are preferably oriented downwardly (as seen best in Fig. 4C) towards the sponge 245 so that when a source of negative pressure is applied to the suction conduit 247, that negative pressure is applied to the wound or ulcer via the sponge 245. As a result, exudate can be wicked and suctioned away from the wound or ulcer via the sponge 245 and into the conduit 247. As shown in Fig. 4A, the bifurcated distal end 249 of the suction conduit 247 extends only partially around the periphery of the unit 230. The proximal end of the conduit may couple to a bottle or other reservoir (not shown) which is also coupled to a source of negative pressure S (shown in Fig. 1). Alternatively, the proximal end of the conduit may terminate in a fluid coupling 259 which in turn can be coupled to an elongate conduit (extension tube) which is coupled to the bottle or reservoir and source of negative pressure. In this manner the conduit may be easily detached from the unit so that the patient can walk or be transported away from the source of suction while pressure is still being applied to the wound or ulcer.

The suction conduit 247 is held between the fluid-filled member 238 and the sponge 245 preferably by an adhesive (not shown) which is applied to the top and bottom of the distal end 249 of the conduit 247, and which binds the distal end 249 to the sponge 245 on its bottom and to the fluid-filled member 238 on its top. Alternatively, the suction conduit 247 may be heat sealed or laser-sealed to the fluid-filled member 238 and/or the sponge 245. In embodiments where the sponge 245 is removable from the fluid-filled member 238, the conduit 247 is preferably adhered only to the bottom of the fluid-filled member 238.

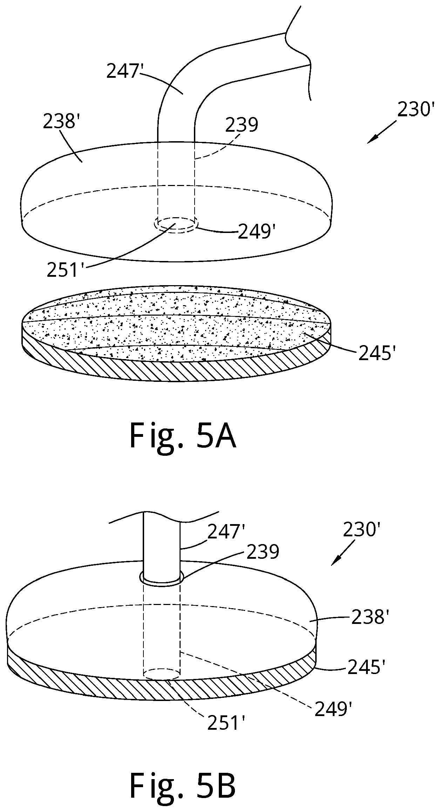

Another embodiment of a unit incorporating a pre-filled air bladder, a sponge and a suction conduit (corresponding to bladder B, foam, sponge or dressing C and suction conduit D of Fig. 1) is seen in Figs. 5A-5B. The primary difference between unit 230' of Figs. 5A-5B and unit 230 of Figs. 4A-4C is that the fluid-filled member 238' defines a central hole 239 (i.e., it is shaped like a donut), and the distal end 249' of the suction conduit 247' is not bifurcated and does not include a series of holes, but rather extends through the central hole of the fluid-filled member 238' and has an open end 251' located at sponge 245'. Preferably, the suction conduit 247' is held in place in the central hole 239 by adhesive (not shown) which binds the distal end 249' of the suction conduit 247' to the walls of the fluid-filled member 238' which define the central hole 239. Alternatively, the suction conduit 247' can be held in place by friction or by other means such that it can be detached from the pre-filled air bladder and sponge unit. As with the embodiment of Figs. 4A-4C, the sponge and fluid-filled member may be strongly or lightly adhered to each other, or the sponge may be removable from a flange element of the fluid-filled member which holds the sponge. In addition, the sponge may be circumferentially sealed.

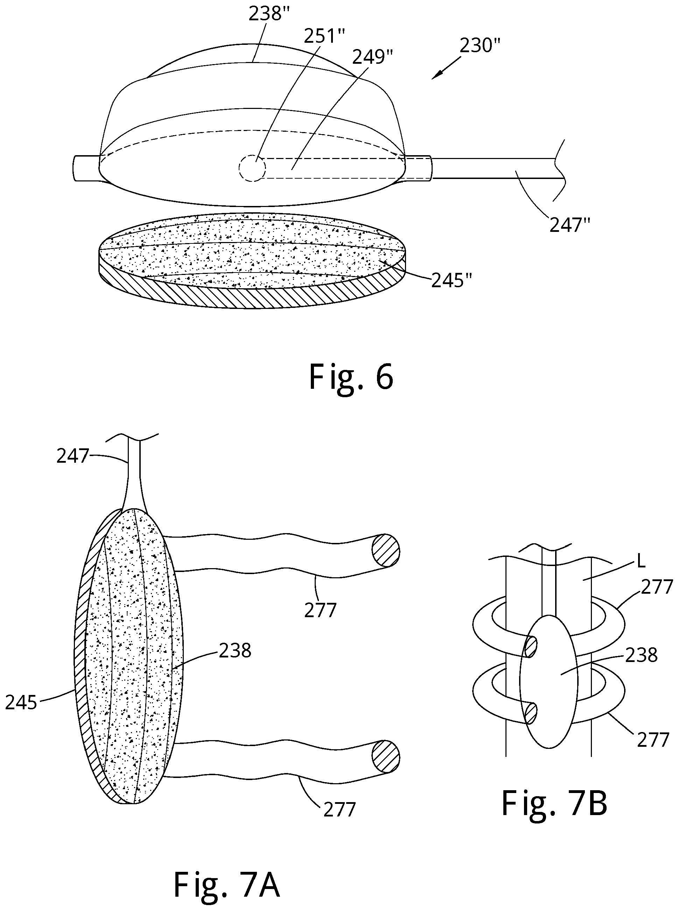

A third embodiment of a unit including a pre-filled air bladder, a sponge, and a suction conduit (corresponding to bladder B, foam, sponge or dressing C and suction conduit D of Fig. 1) is seen in Fig. 6. Unit 230" is similar to units 230 and 230', except that the suction conduit 247" is oriented horizontally between the fluid-filled member 238" and the sponge 245" and includes a distal end 249" having an open end 251". The open end 251" is preferably centrally located in the unit 230", and preferably is oriented in a downward direction (i.e., the top half of the conduit 247" extends further than the bottom half). The suction conduit 247" is held between the fluid-filled member 238" and the sponge 245" preferably by an adhesive (not shown) which is applied to the top and bottom of the distal end 249" of the conduit 247", and which binds the distal end 249" to the sponge 245" on its bottom and to the fluid-filled member 238" on its top. In embodiments where the sponge 245" is removable from the fluid-filled member 238", the conduit 247" is preferably adhered only to the bottom of the fluid-filled member 238". If desired, the sponge 245" may be circumferentially sealed.

In accord with one aspect of the invention, the units 230, 230' and 230" may be provided with additional means for holding the unit in place over the wound or ulcer. Thus, the periphery of the unit might be provided with a biocompatible adhesive. The adhesive could be provided on the sponge, or the fluid-filled member might be provided with a flange (not shown) having a biocompatible adhesive. Alternatively, and as shown in Figs. 7A and 7B, straps 277 may be provided as the means for holding or securing the unit in place. In one embodiment, the straps are formed from the same material and integrally with the fluid-filled member 238, 238', 238" and extend from one side of the fluid-filled member. The straps are adapted to be sufficiently long so as to encircle the limb L. The ends of the straps are provided with an adhesive which is used to couple the straps to the other side of the fluid-filled member. Alternatively, the fluid-filled member may be provided with slots (not shown) into which the straps may be strapped for tightening. In another embodiment, the straps are separately formed and attached to the fluid filled member. Again, the ends of the straps may be provided with an adhesive for coupling the straps to the other side of the fluid-filled member. Alternatively, the fluid-filled member may be provided with slots (not shown) into which the separately formed straps may be strapped for tightening.

In use, a practitioner will select a unit 230, 230', 230", and locate the unit sponge face down over a wound or ulcer. If the unit includes straps, the unit can be strapped in place on the limb. Alternatively, if the unit includes an adhesive ring, the unit is located such that the adhesive ring is preferably located on healthy skin as opposed to the wound or ulcer. Once the unit is located, an appropriate compression mechanism 10, 10', 10", 110 is wrapped over the limb with the air chamber 14, 14', 14", 114 located over the unit 230, 230', 230". When desired, the air chamber is pressurized (or pressure is applied by fixation of the wrap) so that pressure is applied by the compression mechanism to the limb and to the unit. Application of pressure to the unit applies specific pressure to the wound or ulcer and aids in the healing process. Also, when desired, suction is applied to the wound or ulcer by applying a source of negative pressure via the suction tube 247, 247', 247" to the unit. Where the sponge is circumferentially sealed, the amount of suction required to move exudates is lessened. The suction and pressure may be applied together or alone (alternatingly), or one or the other may be constantly applied and the other turned on and off as needed. When applied together, positive mechanical pressure is applied to the wound or ulcer by the compression mechanism and the unit, while negative air pressure is applied to the wound or ulcer via the sponge by the source of negative pressure, thereby removing exudates and further aiding in the healing process. In addition, if desired, a pressure sensor (not shown) may be incorporated with unit 230, 230', 230" and electrically coupled to the suction source S (typically via a wire - not shown - running along suction tube 247, 247', 247"), where the suction source S has an on/off control (not shown). In this manner, the source S of negative pressure can be turned on and off as a function of the mechanical pressure applied by the compression mechanism and the unit as sensed by the sensor (not shown). Thus, if the pressure is above a desired threshold, the suction can be turned on, and if the pressure is below that or a lower threshold the suction can be turned off; or if desired, if the pressure is above a desired threshold the suction can be turned off, and if the pressure is below that or a lower threshold the suction can be turned on. The pressure sensor (not shown) can be located between the fluid-fiilled member 238, 238', 238" and the sponge 245, 245', 245", or in the sponge, or on a surface of the fluid-filled member. Alternatively, the pressure sensor (not shown) can be associated with the air chamber 14, 114, 14', 14" of the flexible member 12, 112, 12',12".

Where the sponge is separable from the fluid-filled chamber, the sponge may be replaced at desired intervals. If not separable, the entire unit may be replaced as desired. Where the unit has a fluid coupling, the suction tube may be detached prior to replacement of the unit.

There have been described and illustrated herein several embodiments of assemblies
of using the same. While particular embodiments of the invention have been described, it is not intended that the invention be limited thereto, as it is intended that the invention be as broad in scope as the art will allow and that the specification be read likewise. Thus, while particular embodiments of compression devices have been disclosed, it will be appreciated that other compression devices could be used as well. In addition, while particular types of units incorporating a fluid-filled bladder, a sponge, and a suction tube have been disclosed, it will be understood that other types of units can be used. For example, and not by way of limitation, the suction device could be a tube which is not bifurcated, but only extends to the edge of the unit; or, the tube could extend around the entire periphery; or, the tube could extend partially or completely around the periphery and also into the center, etc. It will therefore be appreciated by those skilled in the art that yet other modifications could be made to the provided invention

## Claims

1. An assembly (1) comprising:
a) a pressure mechanism (10) having a flexible member (12) for attachment around a limb and an air chamber which assumes a first depressurized state and a second pressurized state, said air chamber having a length and a width, said width being less than half the width of said flexible member;
b) a pre-filled air bladder (14) having a length and a width smaller than said width of said air chamber;
c) an absorbent foam, sponge or dressing (245) coupled to the pre-filled air bladder; and
d) a suction conduit (247) in fluid communication with the absorbent foam, sponge or dressing (245) and adapted for coupling to a source of negative pressure;
wherein:
said pre-filled air bladder (14) and said absorbent foam, sponge or dressing (245) are adhered together as a unit, wherein said suction conduit is located between said absorbent foam, sponge or dressing (245) and said pre-filled air bladder.

2. An assembly according to claim 1, wherein:
said suction conduit (247) terminates on one end in a fluid connector; or
said suction conduit (247) terminates on one end in a bifurcated tube (249) having a plurality of holes (251).

3. An assembly according to claim 2, wherein:
said bifurcated tube (249) extends at least partially around a periphery of said absorbent foam, sponge, or dressing (245).

4. An assembly according to any previous claim, wherein:
said pre-filled air bladder (14) defines a central opening, and said suction conduit (247) extends into said central opening; or
said pre-filled air bladder (14) has straps (125).

5. An assembly according to Claim 1, wherein said prefilled air bladder (14) has a periphery provided with a biocompatible adhesive.

6. An assembly according to Claim 1, wherein:
said absorbent foam, sponge or dressing (247) has a sealed circumference.

## Patentansprüche

1. Anordnung (1), umfassend:
a) einen Druckmechanismus (10) mit einem flexiblen Element (12) zur Befestigung um ein Glied und eine Luftkammer, die einen ersten drucklosen Zustand und einen zweiten druckbeaufschlagten Zustand annimmt, wobei die Luftkammer eine Länge und eine Breite aufweist, wobei die Breite weniger als die Hälfte der Breite des flexiblen Elements beträgt;
b) eine vorgefüllte Luftblase (14) mit einer Länge und einer Breite, die kleiner ist als die Breite der Luftkammer;
c) einen absorbierenden Schaum, Schwamm oder Verband (245), der an die vorgefüllte Luftblase gekoppelt ist; und
d) eine Absaugleitung (247) in Fluidverbindung mit dem absorbierenden Schaum, Schwamm oder Verband (245), und die zur Kopplung an eine Unterdruckquelle ausgelegt ist;
wobei:
die vorgefüllte Luftblase (14) und der absorbierende Schaum, Schwamm oder Verband (245) als eine Einheit zusammengeklebt sind, wobei die Absaugleitung zwischen dem absorbierenden Schaum, Schwamm oder Verband (245) und der vorgefüllten Luftblase angeordnet ist.

2. Anordnung nach Anspruch 1, wobei:
die Absaugleitung (247) an einem Ende in einem Fluidverbinder endet; oder
die Absaugleitung (247) an einem Ende in einem gegabelten Rohr (249) mit einer Vielzahl von Löchern (251) endet.

3. Anordnung nach Anspruch 2, wobei:
sich das gegabelte Rohr (249) mindestens teilweise um einen Umfang des absorbierenden Schaums, Schwamms oder Verbands (245) erstreckt.

4. Anordnung nach einem der vorhergehenden Ansprüche, wobei:
wobei die vorgefüllte Luftblase (14) eine zentrale Öffnung definiert und sich die Absaugleitung (247) in die zentrale Öffnung erstreckt; oder
die vorgefüllte Luftblase (14) Bänder (125) aufweist.

5. Anordnung nach Anspruch 1, wobei die vorgefüllte Luftblase (14) einen Umfang aufweist, der mit einem biokompatiblen Klebstoff versehen ist.

6. Anordnung nach Anspruch 1, wobei:
der absorbierende Schaum, Schwamm oder Verband (247) einen abgedichteten Umfang hat.

## Revendications

1. Ensemble (1) comprenant :
a) un mécanisme de pression (10) comportant un élément souple (12) à fixer autour d'un membre et une chambre à air qui prend un premier état dépressurisé et un second état pressurisé, ladite chambre à air ayant une longueur et une largeur, ladite largeur étant inférieure à la moitié de la largeur dudit élément souple ;
b) une vessie d'air préremplie (14) ayant une longueur et une largeur inférieure à ladite largeur de ladite chambre à air ;
c) une mousse absorbante, une éponge ou un pansement (245) couplé à la vessie d'air préremplie ; et
d) un conduit d'aspiration (247) en communication fluide avec la mousse absorbante, l'éponge ou le pansement (245) et conçu pour se coupler à une source de pression négative ;
dans lequel :
ladite vessie d'air préremplie (14) et ledit mousse absorbante, éponge ou pansement (245) sont collés ensemble en une unité, dans lequel ledit conduit d'aspiration est situé entre ledit mousse absorbante, éponge ou pansement (245) et ladite vessie d'air préremplie.

2. Ensemble selon la revendication 1, dans lequel :
ledit conduit d'aspiration (247) se termine à une extrémité par un raccord de fluide ; ou
ledit conduit d'aspiration (247) se termine à une extrémité par un tube bifurqué (249) ayant une pluralité de trous (251).

3. Ensemble selon la revendication 2, dans lequel :
ledit tube bifurqué (249) s'étend au moins partiellement autour d'une périphérie dudit mousse absorbante, éponge ou pansement (245).

4. Ensemble selon l'une quelconque des revendications précédentes, dans lequel :
ladite vessie d'air préremplie (14) définit une ouverture centrale et ledit conduit d'aspiration (247) s'étend dans ladite ouverture centrale ; ou
ladite vessie d'air préremplie (14) comporte des sangles (125).

5. Ensemble selon la revendication 1, dans lequel ladite vessie d'air préremplie (14) présente une périphérie pourvue d'un adhésif biocompatible.

6. Ensemble selon la revendication 1, dans lequel :
ledit mousse absorbante, éponge ou pansement (247) présente une circonférence scellée.
